# EUROPEAN PATENT SPECIFICATION

(11) **EP 2 175 839 B1**
(45) Date of publication and mention of the grant of the patent: **19.12.2012**
(21) Application number: 08788804.6
(22) Date of filing: 22.07.2008
(51) Int. Cl.: A61K 9/20, A61K 9/50, A61K 31/4453

(54) **CONTROLLED RELEASE PHARMACEUTICAL COMPOSITION OF TOLPERISONE HYDROCHLORIDE**
PHARMAZEUTISCHE ZUSAMMENSETZUNG AUS TOLPERISON-HYDROCHLORID MIT GESTEUERTER FREISETZUNG
COMPOSITION PHARMACEUTIQUE À LIBÉRATION CONTRÔLÉE DE CHLORHYDRATE DE TOLPÉRISONE

(30) Priority: 23.07.2007 HU 0700485
(43) Date of publication of application: 21.04.2010
(73) Proprietor: Richter Gedeon Nyrt., 1103 Budapest (HU)
(72) Inventor: ANTAL, Istvan, 1165 Budapest (HU); KISS, Dorottya, 1203 Budapest (HU); ORGOVAN, Gabor, 2364 Ocsa (HU); STIEDL, Bernadett, 8136 Lajoskomarom (HU); ZELKO, Romana, 1015 Budapest (HU); KLEBOVICH, Imre, 1145 Budapest (HU); NOSZAL, Béla, 1124 Budapest (HU)
(74) Representative: HOFFMANN EITLE
(86) International application number: PCT/HU2008/000086
(87) International publication number: WO 2009/013552

(56) References cited:
- WO-A-00/59508
- WO-A-2005/084676
- WO-A-2005/094825
- WO-A-2007/002238
- WO-A-2007/113856
- GB-A- 1 480 175

## Description

The present invention relates to tolperison hydrochloride containing pharmaceutical compositions with controllable release of active agent and to a process for the preparation thereof.

The controlled release pharmaceutical composition according to the invention comprises multiple granule cores formed with a natural anionic polymer and a lipophil excipient, a hydrophilic matrix-forming excipient which surrounds the cores, and other pharmaceutically acceptable excipients.

Tolperisone is a central muscle relaxant with significant pharmacological effects.

An emphasized pharmacokinetic property of the tolperisone active agent is the excellent absorption from the ileum upon oral administration wherein the maximum plasma concentration (Cmax) is attained within 0,5-1 hour of administration, however the bioavailability is about 20% owing to the significant first-pass metabolism.

Since tolperisone dosing is usually one tablet of three times a day a "once-daily" sustained-release formulation is desirable which provides the daily dose of 450 mg tolperisone hydrochloride active agent by administration once a day.

Numerous patent applications and citations deal with the preparation of retard tolperisone-containing pharmaceutical preparations.

HU patent specification No. 208 920 (Sinnreich) discloses a covered solid retard pharmaceutical composition wherein after oral administration of a dose the composition remains in the stomach providing a continuous release of the active agent while stomach purging occurs periodically. Said composition contains a hydrophilic swelling component and sodium hydrogen carbonate releasing carbon dioxide gas. The above components are surrounded with a membrane (polyvinyl alcohol, fatty acid ester of polyethylene glycol). However, the carbon dioxide gas generation both depends on pH value in the stomach and on the other hand, it is disadvantageous from the physiological point of view.

HU published patent application No. P0200669 discloses a process for the preparation of a prolonged-action oral composition containing tolperisone or a pharmaceutically acceptable salt thereof. In this citation four methods are described for the preparation of retard compositions. According to a method the active agent is covered with a mixture of methanol and a solution of a synthetic, semi-synthetic or natural hydrogel in chloroform in a column under air flow; or microcapsules containing tolperisone are suspended in an aqueous solution saturated with tolperisone. According to a further method water is added to a hydrogel then the mixture is granulated, dried and a mass is formed by mixing the granules with tolperisone and the mixture obtained is diluted with a solution of hydrogel in ethanol. The mass thus obtained is granulated, then mixed with tabletting excipients and tablets are pressed. According to the fourth method tolperisone, lactose, and hydrogel are granulated with an aqueous solution. The granular material obtained is sieved with magnesium stearate and fine silica then homogenized and pressed into tablets. The drawback of the above fourth process is that using of chloroform is unbeneficial to health.

WO 2005/084676, WO 2005/094825 and WO 2005/094676 published international patent applications (Sanochemie) disclose a controlled release coating comprising Eudragit type polymers. The disadvantage of the procedure is the out-of-date tabletting method using organig solvent.

WO 2007/002238 describes drug/polymer composite materials that overcome the disadvantages of using organic solvents by contacting a drug/polymer mixture under pressure with a densified gas solvent, but no sustained release of active ingredients is described.

Our aim was to provide a method for the preparation of a retard pharmaceutical composition containing tolperisone hydrochloride by which disadvantages of the processes described in the technical literature are eliminated while advantages of the known solutions are retained.

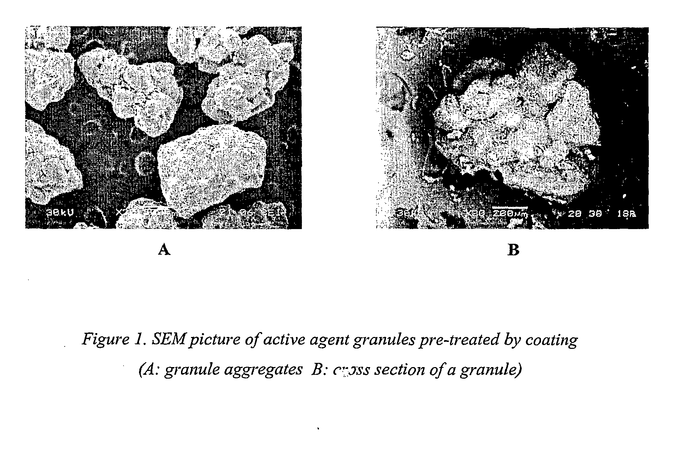

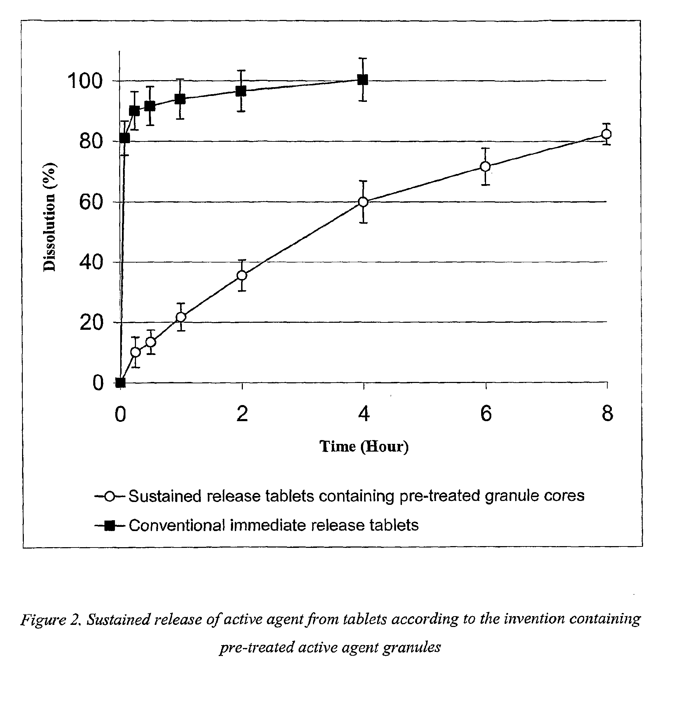

In the course of our experiments granules coated with hydrogel are embedded to a lipophil excipient. The agglomerates obtained are shown in Figure 1. In the granule system obtained the granules of the active agent are adhered and partly coated by a gel formed from the anionic polymer. A lipophil layer is applied onto the granule system by which the granules are further adhered and embedded. The granule system formed by the hydrogel coating has a particle size range of 100-800 µm, preferably 200-600 µm. After applying the lipophil layer the particle size of granule system obtained ranges from 200 to 1200 µm, preferably between 300 and 1000 µm. The granule cores embedded in the lipophil excipient can be dispersed in the hydrophilic matrix-forming excipient of the solid dose form. We have surprisingly found that by using lipophil and hydrophilic excipients simultaneously, and by pre-treating the active agent, a new controlled release system was obtained which provides simultaneously the dissolution sustaining effects of lipophil excipients and the diffusion restraining effects of hydrophilic swelling excipients. It can be seen in Figure 2 that tablets according to the invention show an extended release of the active agent. The release tests were carried out using a Hanson SR8 Type apparatus in 500 ml of release medium, at a temperature of 37±1°C

pH 1,2. The release medium was stirred with rotors at a speed of 50 rpm. The active agent contents of the samples taken in pre-determined points of time were measured by spectrophotometric analysis (260 nm).

The pharmaceutical composition according to the invention provides controlled release of tolperison hydrochloride in the stomach and/or in upper part of the ileum. Beside its advantageous pharmacokinetic profile and extended release effect, a further advantage of the pharmaceutical composition according to the invention is the organic carbonic acid content. Owing to the carbonic acid content the chemical decomposition of the active agent is slowed down. The organic acid, preferably citric acid is present at levels ranging from 5 to 15 w% of the total weight of the composition.

The composition according to the invention exhibits the following dissolution profile: at least 20 % but no more than 60 % of the active agent is released from the composition within 2 hours of administration; at least 30 % but no more than 70 % of the active agent is released from the composition within 4 hours of administration; at least 40 % but no more than 85 % of the active agent is released from the composition within 6 hours of administration; and at least 50 % of the active agent is released from the composition within 8 hours of administration.

The invention relates to a pharmaceutical composition for controlled release of tolperison hydrochloride which comprises multiple granule cores formed with a natural anionic polymer and a lipophil excipient, a hydrophilic matrix-forming excipient which surrounds the cores, and other pharmaceutically acceptable ingredients.

In the pharmaceutical composition according to the invention the natural anionic polymer is sodium alginate or alginic acid, and the natural anionic polymer is present in an amount between 0.1-5 w% of the total weight of the composition, the particle size of the granular core system ranges between 100 and 800 µm, more preferably between 200 and 600 µm.
In the pharmaceutical composition according to the invention, the lipophil excipient is glyceryl palmitostearate or glyceryl behenate.
In a preferred embodiment of the invention the lipophil excipient is present in the composition at levels ranging from 5 to 50 w%, preferably from 10 to 30 w% based on the total weight of the composition. After coating with the lipophil excipient the particle size of the granule system is between 200 and 1200 µm, preferably between 300 and 1000 µm.

In the pharmaceutical composition according to the invention the hydrophilic matrix-forming excipient being present in the outer phase, owing to its low density after swelling provides "floating" properties to the composition.

The hydrophilic matrix-forming excipient, which surrounds the cores of the pharmaceutical composition is polyethylene oxide.

In a preferred embodiment of the invention the active agent of the pharmaceutical composition is homogenized with an organic acid, preferably with 5-15 w/w% of citric acid. The present invention further involves a method for the preparation of a controlled release pharmaceutical composition containing tolperison hydrochloride which comprises the following steps: active agent is granulated with the colloidal solution of the above natural anionic polymer free from organic solvents under 4.5 pH value, the granular core obtained is coated with the melt of the above lipophil excipient or with any kind of its physical mixture and it is granulated, then the coated granule cores thus obtained are mixed with the above matrix-forming excipient and with other excipients and the resulting mixture is pressed into tablets.

The present invention is now explained in detail by the following examples, which are given to illustrate the invention rather than to limit the scope.

### Example 1 (1000 tablets)

In the following Example 1 composition and manufacture of a pharmaceutical preparation according to the invention are described.

| **Ingredients** | **Weight(g)** | **Amounts in w/w ratios (%)** | **Amounts in final w/w ratios (%)** |
|---|---|---|---|
| *Core:* | | | |
| Tolperison HCl | 450 | 51.72 | 54.95 |
| Sodium alginate | 14 | 1.61 | 1.71 |
| Demineralised water | 56 | 6.44 | |

| *Coat:* | | | |
|---|---|---|---|
| Glyceryl palmitostearate | 140 | 16.09 | 17.09 |

| *Outer phase:* | | | |
|---|---|---|---|
| Polyethylene oxide (high molecular weight) | 100 | 11.49 | 12.21 |
| Microcrystalline cellulose | 100 | 11.49 | 12.21 |
| Magnesium stearate | 10 | 1.15 | 1.22 |

For the preparation of the pre-treated granular cores the active agent is mixed with sodium alginate in a Foucault current granulating machine at a speed of 1000 rpm. The granule cores may be prepared also by granulating the active agent with a swelled sodium alginate gel. The cores are then dried in an exsiccator, or in a tray or fluid dryer at a temperature of 40°C until achieving the desired moisture content, or for 24 hours. In the following steps the granule cores fraction having particle diameters greater than 100 µm are used.

In a Stephan UMC-5 type Foucault current granulating machine the glyceryl palmitostearate is melted by heating it to some degrees over the melting point (58°C). The granule system having a temperature between 2-8°C is poured into the melted glyceryl palmitostearate under stirring. The fraction of the coated cores having particle size between 200 and 1000 µm is mixed with the ingredients of the outer phase in a homogenizer at low rpm. The mixture thus obtained is then compressed into tablets in a Diaf excenter tabletting machine using a 15 mm mould.

### Example 2 (1000 tablets)

In the following Example 2 the composition and manufacture of a sustained release pharmaceutical composition containing granule cores of the pre-treated active agent are described.

| **Ingredients** | **weight (g)** | **Amounts in w/w ratios (%)** | **Amounts in final w/w ratios (%)** |
|---|---|---|---|
| *Core:* | | | |
| Tolperison HCl | 450 | 51.72 | 54.95 |
| Sodium alginate | 14 | 1.61 | 1.71 |
| Demineralised water | 56 | 6.44 | |

| *Coat:* | | | |
|---|---|---|---|
| Glyceryl palmitostearate | 140 | 16.09 | 17.09 |

| *Outer phase:* | | | |
|---|---|---|---|
| Polyethylene oxide (high molecular weight) | 100 | 11.49 | 12.21 |
| Microcrystalline cellulose | 100 | 11.49 | 12.21 |
| Magnesium stearate | 10 | 1.15 | 1.22 |

The pharmaceutical composition according to Example 1 is prepared except that granule cores of the active agent pre-treated with alginate gel are dried after granulation in a Collette type microwave vacuum drier to a moisture content of 1 %.

### Example 3 (1000 tablets)

In the following Example 3 the composition and manufacture of a sustained release pharmaceutical composition are described.

| **Ingredients** | **Weight (g)** | **Amounts in w/w ratios (%)** | **Amounts in final w/w ratios (%)** |
|---|---|---|---|
| *Core:* | | | |
| Tolperison HCl | 450 | 48.91 | 51.78 |
| Citric acid | 50 | 5.43 | 5.75 |
| Sodium alginate | 14 | 1.52 | 1.61 |
| Demineralised water | 56 | 6.09 | |

| *Coat:* | | | |
|---|---|---|---|
| Glyceryl palmitostearate | 140 | 15.22 | 16.11 |

| *Outer phase:* | | | |
|---|---|---|---|
| Polyethylene oxide (high molecular weight) | 100 | 10.87 | 11.51 |
| Microcrystalline cellulose | 100 | 10.87 | 11.51 |
| Magnesium stearate | 10 | 1.09 | 1.15 |

The granular cores are prepared according to Example 1 except that before granulating the active agent cores are homogenized with citric acid.

### Example 4 (1000 tablets)

The following Example 4 describes the composition and manufacture of a sustained release pharmaceutical composition according to the invention.

| **Ingredients** | **Weight (g)** | **Amounts in w/w ratios (%)** | **Amounts in final w/w ratios (%)** |
|---|---|---|---|
| *Core:* | | | |
| Tolperison HCl | 450 | 56.25 | 60.08 |
| Sodium alginate | 14 | 1.75 | 1.87 |
| Demineralised water | 56 | 7.00 | |

| *Coat:* | | | |
|---|---|---|---|
| Glyceryl palmitostearate | 70 | 8.75 | 9.35 |

| *Outer phase:* | | | |
|---|---|---|---|
| Polyethylene oxide (high molecular weight) | 100 | 12.50 | 13.35 |
| Microcrystalline cellulose | 100 | 12.50 | 13.35 |
| Magnesium stearate | 10 | 1.25 | 1.34 |

The procedure described in Example 1 is carried out to prepare granule cores.

### Example 5 (1000 tablets)

Example 5 describes the composition and manufacture of a sustained release pharmaceutical composition according to the invention.

| **Ingredients** | **Weight (g)** | **Amounts in w/w ratios (%)** | **Amounts in final w/w ratios (%)** |
|---|---|---|---|
| *Core:* | | | |
| Tolperison HCl | 450 | 44.55 | 46.92 |
| Sodium alginate | 14 | 1.39 | 1.46 |
| Demineralised water | 56 | 5.54 | |

| *Coat:* | | | |
|---|---|---|---|
| Glyceryl palmitostearate | 280 | 27.72 | 29.20 |

| *Outer phase:* | | | |
|---|---|---|---|
| Polyethylene oxide (high molecular weight) | 100 | 9.90 | 10.43 |
| Microcrystalline cellulose | 100 | 9.90 | 10.43 |
| Magnesium stearate | 10 | 0.99 | 1.04 |

The procedure according to Example 1 is carried out to prepare the granule cores.

### Example 6 (1000 tablets):

Example 6 describes the composition and manufacture of a sustained release pharmaceutical composition according to the invention.

| **Ingredients** | **Weight (g)** | **Amounts in w/w ratios (%)** | **Amounts in final w/w ratios (%)** |
|---|---|---|---|
| *Core:* | | | |
| Tolperison HCl | 450 | 42.06 | 44.16 |
| Sodium alginate | 14 | 1.31 | 1.37 |
| Demineralise water | 56 | 5.23 | |

| *Coat:* | | | |
|---|---|---|---|
| Glyceryl palmitostearate | 140 | 13.08 | 13.74 |

| *Outer phase:* | | | |
|---|---|---|---|
| Polyethylene oxide (high molecular weight) | 300 | 28.04 | 29.44 |
| Microcrystalline cellulose | 100 | 9.35 | 9.81 |
| Magnesium stearate | 10 | 0.93 | 0.98 |

The preparation is manufactured according to Example 1 except, that the outer phase contains more polyethylene oxide to attain the "floating" property. After swelling, the polyethylene oxide owing to its low density, hydrodynamically equalizes the pharmaceutical product. The "floating" property means that the tablet floats on the surface of the gastric juice after 5 minutes of administration and this floating remains for 8 hours of the release test.

### Example 7 (1000 tablets)

The following Example 7 describes the composition and manufacture of a sustained release pharmaceutical composition according to the invention.

| **Ingredients** | **Weight (g)** | **Amounts in weight ratios (%)** | **Amounts in final weight ratios (%)** |
|---|---|---|---|
| *Core:* | | | |
| Tolperison HCl | 450 | 51.72 | 54.95 |
| Sodium alginate | 14 | 1.61 | 1.71 |
| Demineralised water | 56 | 6.44 | |

| *Coat:* | | | |
|---|---|---|---|
| Glyceryl palmitostearate | 140 | 16.09 | 17.09 |

| *Outer phase:* | | | |
|---|---|---|---|
| Polyethylene oxide (low molecular weight) | 100 | 11.49 | 12.21 |
| Microcrystalline cellulose | 100 | 11.49 | 12.21 |
| Magnesium stearate | 10 | 1.15 | 1.22 |

The procedure described in Example 1 is carried out to prepare the granule cores.

### Example 8 (1000 tablets)

The following Example 8 describes the composition and manufacture of a sustained release pharmaceutical composition according to the invention.

| **Ingredients** | **Weight (g)** | **Amounts in weight ratios (%)** | **Amounts in final weight ratios (%)** |
|---|---|---|---|
| *Core:* | | | |
| Tolperison HCl | 450 | 51.72 | 54.95 |
| Sodium alginate | 14 | 1.61 | 1.71 |
| Demineralised water | 56 | 6.44 | |

| *Coat:* | | | |
|---|---|---|---|
| Gliceryl palmitostearate | 140 | 16.09 | 17.09 |

| *Outer phase :* | | | |
|---|---|---|---|
| Polyethylene oxide (high molecular weight) | 50 | 5.75 | 6.11 |
| Polyethylene oxide (low molecular weight) | 50 | 5.75 | 6.11 |
| Microcrystalline cellulose | 100 | 11,49 | 12.21 |
| Magnesium stearate | 10 | 1.15 | 1.22 |

The granule cores are prepared according to the procedure described in Example 1.

### Example 9 (1000 tablets)

The following example 9 describes the composition and manufacture of a sustained release pharmaceutical composition according to the invention.

| **Ingredients** | **Weight (g)** | **Amounts in weight ratios (%)** | **Amounts in final weight ratios (%)** |
|---|---|---|---|
| *Core:* | | | |
| Tolperison HCl | 450 | 51.72 | 54.95 |
| Sodium alginate | 14 | 1.61 | 1.71 |
| Demineralised water | 56 | 6.44 | |

| *Coat:* | | | |
|---|---|---|---|
| Glyceryl palmitostearate | 140 | 16.09 | 17.09 |

| *Outer phase:* | | | |
|---|---|---|---|
| Polyethylene oxide (high molecular weight) | 100 | 11.49 | 12.21 |
| Lactose monohydrate | 100 | 11.49 | 12.21 |
| Magnesium stearate | 10 | 1.15 | 1.22 |

The granule cores are prepared according to the procedure described in Example 1.

### Example 10 (1000 capsules):

The following Example 10 describes the manufacture of a sustained release pharmaceutical composition containing pre-treated granules of the active agent.

| **Ingredients** | **Weight (g)** | **Amounts in weight ratios (%)** | **Amounts in final weight ratios (%)** |
|---|---|---|---|
| *Core:* | | | |
| Tolperison HCl | 450 | 58.44 | 62.59 |
| Sodium alginate | 14 | 1.82 | 1.95 |
| Demineralised water | 56 | 7.27 | |

| *Coat:* | | | |
|---|---|---|---|
| Glyceryl palmitostearate | 140 | 18.18 | 19.47 |

| *Outer phase:* | | | |
|---|---|---|---|
| Polyethylene oxide (high molecular weight) | 100 | 12.99 | 13.91 |
| Magnesium stearate | 10 | 1.30 | 1.39 |

The composition is prepared according to Example 1 with the difference that particle size of the pre-treated active agent coated with glyceryl palmitostearate is within a wider range of 500-800 µm. The coated granules obtained wherein the particle size ranges from 800 and 1200 µm are filled into 000 size hard gelatine capsules.

### Example 11 (1000 capsules):

The Example 11 describes the composition and manufacture of a sustained release pharmaceutical composition according to the invention.

| **Ingredients** | **Weight (g)** | **Amounts in weight ratios (%)** | **Amounts in final weight ratios (%)** |
|---|---|---|---|
| *Core:* | | | |
| Tolperison HCl | 450 | 51.72 | 54.95 |
| Sodium alginate | 14 | 1.61 | 1.71 |
| Demineralised water | 56 | 6.44 | |

| *Coat:* | | | |
|---|---|---|---|
| Glyceryl behenate | 140 | 16.09 | 17.09 |

| *Outer phase:* | | | |
|---|---|---|---|
| Polyethylene oxide (high molecular weight) | 100 | 11.49 | 12.21 |
| Microcrystalline cellulose | 100 | 11.49 | 12.21 |
| Magnesium stearate | 10 | 1.15 | 1.22 |

The granule cores are prepared according to the procedure described in Example 1.

## Claims

1. A controlled release pharmaceutical composition for administration of tolperison hydrochloride comprising multiple granule cores formed with a natural anionic polymer being alginic acid or sodium alginate and a lipophil excipient selected from the group consisting of glyceryl palmitostearate and glyceryl behenate, polyethylene oxide as hydrophilic matrix-forming excipient which surrounds the cores, and other pharmaceutically acceptable excipients.

2. The controlled release pharmaceutical composition of claim 1, wherein the natural anionic polymer is applied in 0.1-5 wt% based on the total weight of the composition.

3. The controlled release pharmaceutical composition of claim 1 or claim 2, wherein the particle size of granule system formed with the natural anionic polymer is in the range of 100-800 µm.

4. The controlled release pharmaceutical composition of claim 1, wherein the weight ratio of the lipophil excipient to the total weight of the composition is between 5-50 w/w%.

5. The controlled release pharmaceutical composition of claim 1, wherein the weight ratio of the lipophil excipient to the total weight of the composition is between 10-30 w/w%.

6. The controlled release pharmaceutical composition of claim 5, wherein the particle size of the granule system after coating with the lipophil excipient is in the range of 200-1200 µm.

7. The controlled release pharmaceutical composition of any one of claims 1-6, wherein the composition further contains an organic acid.

8. The controlled release pharmaceutical composition of claim 7, wherein the composition contains citric acid as organic acid.

9. The controlled release pharmaceutical composition of claim 8, wherein the amount of citric acid is in the range of 5-15 wt% of total weight of the composition.

10. Process for the preparation of a pharmaceutical composition of any one of claims 1-9 comprising the following steps: active agent is granulated with colloidal solution of a natural anionic polymer being alginic acid or sodium alginate, free from organic solvents under pH value of 4.5, the granular core obtained is coated with the melt of a lipophil excipient selected from the group consisting of glyceryl palmitostearate and glyceryl behenate, or with any kind of its physical mixture and it is granulated, then the coated granule cores obtained are mixed with polyethylene oxide as matrix-forming excipient and other excipients and the resulting mixture is pressed into tablets.

## Patentansprüche

1. Kontrolliert freisetzende pharmazeutische Zusammensetzung zur Verabreichung von Tolperisonhydrochlorid, die mehrere Granulatkerne umfasst, die mit einem natürlichen anionischen Polymer gebildet sind, das Alginsäure oder Natriumalginat ist, und einen lipophilen Hilfsstoff ausgewählt aus der Gruppe bestehend aus Glycerylpalmitostearat und Glycerylbehenat, Polyethylenoxid als Hilfsstoff, der eine hydrophile Matrix bildet und die Kerne umgibt, und andere pharmazeutisch annehmbare Hilfsstoffe umfasst.

2. Kontrolliert freisetzende pharmazeutische Zusammensetzung gemäß Anspruch 1, worin das natürliche anionische Polymer zu 0,1 bis 5 Gew.% auf Basis des Gesamtgesichts der Zusammensetzung eingesetzt wird.

3. Kontrolliert freisetzende pharmazeutische Zusammensetzung gemäß Anspruch 1 oder Anspruch 2, worin die Partikelgröße des Granulatsystems, das mit dem natürlichen anionischen Polymer gebildet ist, im Bereich von 100 bis 800 µm ist.

4. Kontrolliert freisetzende pharmazeutische Zusammensetzung gemäß Anspruch 1, worin das Gewichtsverhältnis des lipophilen Hilfsstoff zum Gesamtgewicht der Zusammensetzung zwischen 5 und 50 G/G% ist.

5. Kontrolliert freisetzende pharmazeutische Zusammensetzung gemäß Anspruch 1, worin das Gewichtsverhältnis des lipophilen Hilfsstoff zum Gesamtgewicht der Zusammensetzung zwischen 10 und 30 G/G% ist.

6. Kontrolliert freisetzende pharmazeutische Zusammensetzung gemäß Anspruch 5, worin die Partikelgröße des Granulatsystems nach dem Beschichten mit dem lipophilen Hilfsstoff im Bereich von 200 bis 1.200 µm ist.

7. Kontrolliert freisetzende pharmazeutische Zusammensetzung gemäß irgendeinem der Ansprüche 1 bis 6, worin die Zusammensetzung weiterhin eine organische Säure enthält.

8. Kontrolliert freisetzende pharmazeutische Zusammensetzung gemäß Anspruch 7, worin die Zusammensetzung Zitronensäure als organische Säure enthält.

9. Kontrolliert freisetzende pharmazeutische Zusammensetzung gemäß Anspruch 8, worin die Menge der Zitronensäure im Bereich von 5 bis 15 Gew.% des Gesamtgewichts der Zusammensetzung ist.

10. Verfahren zur Herstellung einer pharmazeutischen Zusammensetzung gemäß irgendeinem der Ansprüche 1 bis 9, das die folgenden Schritte umfasst: der Wirkstoff wird mit einer kolloidalen Lösung eines natürlichen anionischen Polymers, das Algininsäure oder Natriumalginat ist, frei von organischen Lösungsmitteln unter einem pH-Wert von 4,5 granuliert, der so erhaltene Granulatkern wird mit der Schmelze eines lipophilen Hilfsstoffs ausgewählt aus der Gruppe bestehend aus Glycerylpalmitostearat und Glycerylbehenat oder mit irgendeiner Sorte deren physikalischer Mischung beschichtet und wird granuliert, dann werden die erhaltenen beschichteten Granulatkerne mit Polyethylenoxid als matrixbildenden Hilfsstoff und anderen Hilfsstoffen beschichtet und die resultierende Mischung wird zu Tabletten gepresst.

## Revendications

1. Composition pharmaceutique à libération contrôlée pour l'administration de tolpérison chlorhydrate comprenant de multiples coeurs de granules formés avec un polymère anionique naturel qui est l'acide alginique ou l'alginate de sodium et un excipient lipophile choisi dans le groupe constitué par le palmitostéarate de glycéryle et le béhénate de glycéryle, du poly(oxyde d'éthylène) en tant qu'excipient formant une matrice hydrophile qui entoure les coeurs, et d'autres excipients pharmaceutiquement acceptables.

2. Composition pharmaceutique à libération contrôlée selon la revendication 1, dans laquelle le polymère anionique naturel est appliqué à raison de 0,1 à 5 % en poids par rapport au poids total de la composition.

3. Composition pharmaceutique à libération contrôlée selon la revendication 1 ou la revendication 2, dans laquelle la granulométrie du système de granules formé avec le polymère anionique naturel est située dans la plage allant de 100 à 800 µm.

4. Composition pharmaceutique à libération contrôlée selon la revendication 1, dans laquelle le rapport en poids de l'excipient lipophile au poids total de la composition est compris entre 5 à 50 % en poids/poids.

5. Composition pharmaceutique à libération contrôlée selon la revendication 1, dans laquelle le rapport en poids de l'excipient lipophile au poids total de la composition est compris entre 10 et 30 % en poids/poids.

6. Composition pharmaceutique à libération contrôlée selon la revendication 5, dans laquelle la granulométrie du système de granules après revêtement avec l'excipient lipophile est située dans la plage allant de 200 à 1200 µm.

7. Composition pharmaceutique à libération contrôlée selon l'une quelconque des revendications 1 à 6, laquelle composition contient en outre un acide organique.

8. Composition pharmaceutique à libération contrôlée selon la revendication 7, laquelle composition contient de l'acide citrique en tant qu'acide organique.

9. Composition pharmaceutique à libération contrôlée selon la revendication 8, dans laquelle la quantité d'acide citrique est située dans la plage allant de 5 à 15 % en poids par rapport au poids total de la composition.

10. Procédé pour la préparation d'une composition pharmaceutique de l'une quelconque des revendications 1 à 9, comprenant les étapes suivantes : un agent actif est granulé avec une solution colloïdale d'un polymère anionique naturel qui est l'acide alginique ou l'alginate de sodium, ne contenant pas de solvants organiques sous une valeur de pH de 4,5, le coeur granulaire obtenu est revêtu de la masse fondue d'un excipient lipophile choisi dans le groupe constitué par le palmitostéarate de glycéryle et le béhénate de glycéryle, ou avec n'importe quel type de son mélange physique, et est granulé, puis les coeurs de granules revêtus obtenus sont mélangés avec du poly(oxyde d'éthylène) servant d'excipient formant une matrice et d'autres excipients, et le mélange résultant est pressé en comprimés.
